(19) **Europäisches Patentamt**
**European Patent Office**
**Office européen des brevets**

(11) **EP 2 521 569 B1**

(12) **EUROPEAN PATENT SPECIFICATION**

(45) Date of publication and mention
of the grant of the patent:
**01.11.2017 Bulletin 2017/44**

(21) Application number: **11731736.2**

(22) Date of filing: **05.01.2011**

(51) Int Cl.:
*A61K 45/00* (2006.01)      *A61K 31/685* (2006.01)
*A61P 29/00* (2006.01)

(86) International application number:
**PCT/IL2011/000010**

(87) International publication number:
**WO 2011/083467 (14.07.2011 Gazette 2011/28)**

(54) **COMBINED TREATMENT UTILIZING VB-201**

KOMBINATIONSTHERAPIE MIT VB-201

TRAITEMENT COMBINÉ FAISANT APPEL AU VB-201

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.12.2010 US 423112 P
04.10.2010 US 389318 P
07.06.2010 US 351975 P
07.01.2010 US 282250 P
05.01.2010 US 292226 P**

(43) Date of publication of application:
**14.11.2012 Bulletin 2012/46**

(73) Proprietor: **Vascular Biogenics Ltd.
60376 Or Yehuda (IL)**

(72) Inventors:
• **COHEN, Yael
55602 Kiryat-Ono (IL)**
• **YACOV, Niva
69705 Tel-Aviv (IL)**
• **BREITBART, Eyal
73127 Hashmonaim (IL)**

(74) Representative: **Vossius & Partner
Patentanwälte Rechtsanwälte mbB
Siebertstrasse 3
81675 München (DE)**

(56) References cited:
**US-A1- 2008 261 865      US-A1- 2008 261 865
US-A1- 2009 149 541      US-A1- 2009 197 242**

• **MENDEL I ET AL: "A Lecinoxoid, an oxidized
phospholipid small molecule, constrains CNS
autoimmune disease", JOURNAL OF
NEUROIMMUNOLOGY, ELSEVIER SCIENCE
PUBLISHERS BV, XX, vol. 226, no. 1-2, 14
September 2010 (2010-09-14), pages 126-135,
XP027265555, ISSN: 0165-5728 [retrieved on
2010-09-07]**
• **REAGAN-SHAW S ET AL: "Dose translation from
animal to human studies revisited", FASEB
JOURNAL, FED. OF AMERICAN SOC. FOR
EXPERIMENTAL BIOLOGY, US, vol. 22, 1 January
2007 (2007-01-01), pages 659-661, XP007916336,
ISSN: 0892-6638**

Remarks:
The file contains technical information submitted after
the application was filed and not included in this
specification

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

FIELD AND BACKGROUND OF THE INVENTION

The present invention, in some embodiments thereof, relates to the field of pharmacology and more particularly, to novel dosages, treatment regimens and therapeutic uses of the oxidized phospholipid VB-201.

Oxidized phospholipids have been previously described as useful in the treatment of medical conditions such as, for example, cardiovascular diseases, cerebrovascular diseases and inflammatory diseases and disorders.

International Patent Application No. PCT/IL2004/000453 (Publication No. WO 04/106486), by the present assignee, describes oxidized lipids for prevention and treatment of inflammation associated with endogenous oxidized lipids. An exemplary such compound is described and known as CI-201 (1-hexadecyl-2-(4'-carboxybutyl)-glycerol-3-phosphocholine; also referred to in the art as VB-201).

International Patent Application No. PCT/IL01/01080 (Publication No. WO 02/41827), by the present assignee, describes oxidized lipids for prevention and treatment of atherosclerosis and related diseases.

International Patent Application Nos. PCT/IL2004/000453 and PCT/IL01/01080 describe co-administration of an oxidized lipid with a statin.

Statins, which are also known in the art as HMG-CoA reductase inhibitors, are a class of drugs used to lower cholesterol levels by inhibiting the enzyme HMG-CoA reductase, which plays an important role in the production of cholesterol. Statins are widely administered in order to treat cardiovascular disease, and in order to prevent development of cardiovascular disease in subjects exhibiting risk factors for cardiovascular disease, such as elevated cholesterol levels, diabetes, and/or high blood pressure. Statin administration reduces major coronary events by 27 % to 37 % (vs. placebo) [Lancet 2002, 350:7-22; Lancet 1995, 333:1301-1307; Lancet 1994, 344:1383-1389; Lancet 2003, 361:1149-1157; Lancet 2004,364:685-696].

Statins are associated with a number of adverse side effects, primarily elevated blood levels of liver enzymes and moderate muscle problems (e.g., myalgia, muscle cramps), but also gastrointestinal problems, polyneuropathy, and relatively severe muscle problems such as myositis, myopathy and rhabdomyolysis (which can lead to acute renal failure). In addition, the level of statins in the body is elevated by grapefruit consumption. In view of the toxicity of statins, subjects undergoing statin treatment are advised to avoid grapefruit consumption.

Statins have been administered in combination with fibrates, another class of lipid-lowering drugs. However, such a combined treatment is associated with a significantly elevated risk for muscle problems, including rhabdomyolysis.

Glatiramer acetate (marketed as Copaxone®) is an immunomodulating random polymer of glutamic acid, lysine, alanine and tyrosine, which is used to treat multiple sclerosis. Glatiramer acetate is also effective in experimental models of inflammatory conditions such as colitis and cerebral malaria, and is undergoing clinical trials for treatment of dry age-related macular degeneration.

Additional background art includes International Patent Application Nos. PCT/IL05/000735 (Publication No. WO 06/006161), PCT/IL02/00005 (Publication No. WO 02/053092) and PCT/IL08/000013 (Publication No. WO 08/084472), all being also by the present assignee. Furthermore, US 2008/0261865 describes synthetic oxidized lipids and methods utiliting oxidized lipids for treating and preventing an inflamation associated with an endongenous oxidized liquid.

SUMMARY OF THE INVENTION

The present invention provides the following:

1. VB-201, 1-hexadecyl-2-(4'-carboxybutyl)-glycerol-3-phosphocholine, for use in treating or preventing an inflammatory disease or disorder, wherein a therapeutically effective amount of VB-201 is to be orally administered to a human subject in need thereof, wherein the therapeutically effective amount of VB-201 is a daily dosage of 160 mg, wherein the daily dosage is effected by administering a unit dosage form comprising 40 mg or 80 mg VB-201 multiple times per day at different times during the day.

2. VB-201 for use of item 1, wherein the daily dosage is effected by administering two unit dosage forms, each comprising 80 mg VB-201, at different times during the day.

3. VB-201 for use of item 1 or 2, wherein the inflammatory disease or disorder is

(i) an inflammatory gastrointestinal disease or disorder selected from the group consisting of colitis, ileitis, Crohn's disease, chronic inflammatory intestinal disease, inflammatory bowel syndrome, celiac disease, an ulcer, a skin ulcer, a bed sore, a gastric ulcer, a peptic ulcer, a buccal ulcer, a nasopharyngeal ulcer, an esophageal ulcer, a duodenal ulcer, and a gastrointestinal ulcer,
(ii) an autoimmune disease or disorder selected from the group consisting of chronic rheumatoid arthritis, juvenile rheumatoid arthritis, systemic lupus erythematosus, scleroderma, mixed connective tissue disease, polyarteritis nodosa, polymyositis/dermatomyositis, Sjogren's syndrome, Bechet's disease, multiple sclerosis, autoimmune diabetes, Hashimoto's disease, primary myxedema, pernicious anemia, myasthenia gravis, chronic active hepatitis, autoimmune hemolytic anemia, idiopathic thrombocytopenic purpura, uveitis, vasculitides, and heparin induced thrombocytopenia,
(iii) an inflammatory cardiovascular disease or disorder selected from the group consisting of occlusive diseases or disorders, atherosclerosis, a cardiac valvular disease, stenosis, restenosis, in-stent-stenosis, myocardial infarction, coronary arterial disease, acute coronary syndromes, congestive heart failure, angina pectoris, myocardial ischemia, thrombosis, Wegener's granulomatosis, Takayasu's arteritis, Kawasaki syndrome, anti-factor VIII autoimmune disease or disorder, necrotizing small vessel vasculitis, microscopic polyangiitis, Churg and Strauss syndrome, pauci-immune focal necrotizing glomerulonephritis, crescentic glomerulonephritis, antiphospholipid syndrome, antibody induced heart failure, thrombocytopenic purpura, autoimmune hemolytic anemia, cardiac autoimmunity, Chagas' disease or disorder, and anti-helper T lymphocyte autoimmunity,
(iv) an inflammatory cerebrovascular disease or disorder selected from the group consisting of stroke, cerebrovascular inflammation, cerebral hemorrhage, and vertebral arterial insufficiency, or
(v) a peripheral vascular disease or disorder selected from the group consisting of gangrene, diabetic vasculopathy, ischemic bow el disease, thrombosis, diabetic retinopathy, and diabetic nephropathy.

4. VB-201 for use of item 1 or 2, wherein the inflammatory disease or disorder is colitis.

5. VB-201 for use of item 1 or 2, wherein the inflammatory cardiovascular disease or disorder is atherosclerosis.

6. VB-201 for use of item 1 or 2, wherein the inflammatory cardiovascular disease or disorder is acute coronary syndrome.

7. VB-201 for use of item 1 or 2, wherein the inflammatory cardiovascular disease or disorder is angina pectoris.

8. VB-201 for use of item 1 or 2, wherein the inflammatory cerebrovascular disease or disorder is stroke.

9. VB-201 for use of any one of items 1 to 8, wherein a therapeutically effective amount of glatiramer acetate is to be co-administered with VB-201.

10. VB-201 for use of item 9, wherein the therapeutically effective amount of glatiramer acetate is in a range of from 2-200 mg/day.

11. VB-201 for use of item 1, wherein the inflammatory disease or disorder is a proliferative disease or disorder.

12. VB-201 for use of item 11, wherein the proliferative disease or disorder is cancer.

BRIEF DESCRIPTION OF THE DRAWINGS

The patent or application file contains at least one drawing executed in color. Copies of this patent or patent application publication with color drawing(s) will be provided by the Office upon request and payment of the necessary fee.
Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
In the drawings:

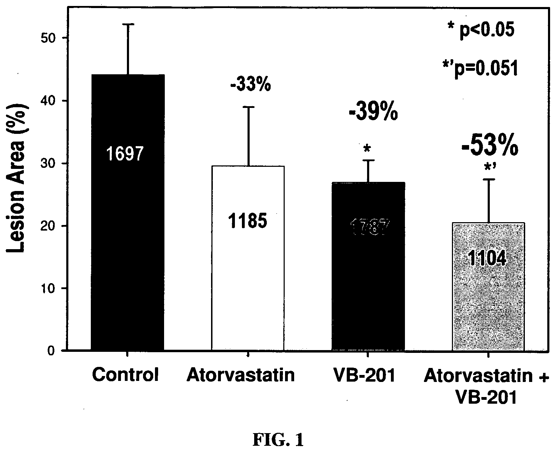

FIG. 1 is a graph showing the effect of 2.5 mg/kg atorvastatin and 4 mg/kg VB-201, alone or in combination, on lesion areas in the aorta of rabbits fed a high-cholesterol diet;

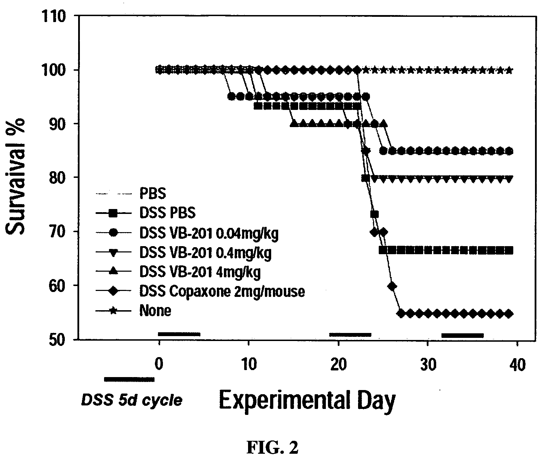

FIG. 2 is a graph showing the survival of mice treated with 0.04, 0.4 or 4 mg/kg VB-201, 2 mg per mouse glatiramer acetate or PBS (with 0.5 % ethanol), as a function of time following induction of colitis by dextran sulfate sodium (DSS); survival of mice treated with PBS or no treatment (none) without receiving DSS is shown as a control;

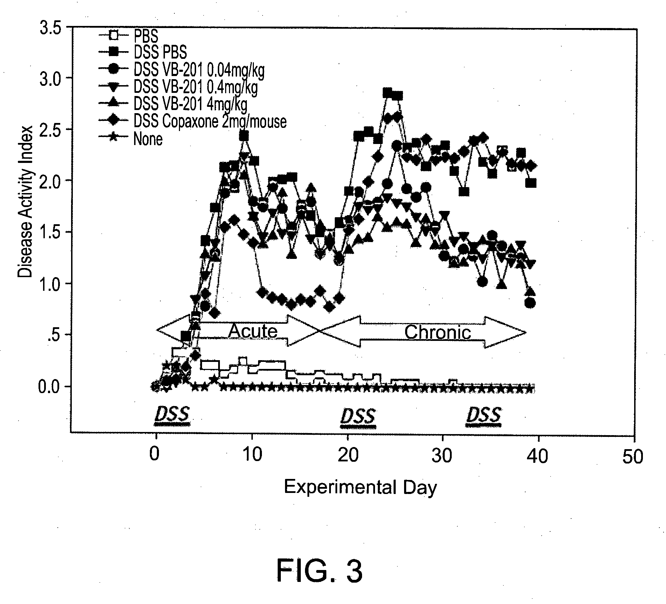

FIG. 3 is a graph showing the disease activity index (DAI) of mice treated with 0.04, 0.4 or 4 mg/kg VB-201, 2 mg per mouse glatiramer acetate or PBS (with 0.5 % ethanol), as a function of time following induction of colitis by DSS; DAI of mice treated with PBS or no treatment (none) without receiving DSS is shown as a control (experimental time period is divided into acute and chronic phases of the disease);

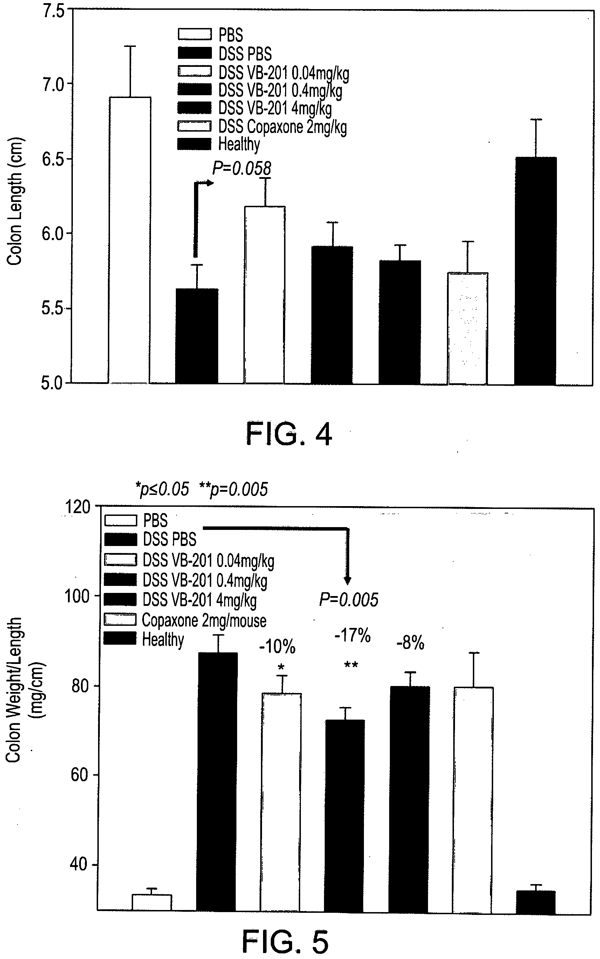

FIG. 4 is a graph showing the colon length of mice treated with 0.04, 0.4 or 4 mg/kg VB-201, 2 mg per mouse glatiramer acetate or PBS (with 0.5 % ethanol) 39 days following induction of colitis by DSS; colon length of mice treated with PBS or no treatment (healthy) without receiving DSS is shown as a control (p=0.058 for 0.04 mg/kg VB-201 relative to PBS treatment in mice receiving DSS);

FIG. 5 is a graph showing the colon weight/length ratio of mice treated with 0.04, 0.4 or 4 mg/kg VB-201, 2 mg per mouse glatiramer acetate or PBS (with 0.5 % ethanol) 39 days following induction of colitis by DSS; colon weight/length ratio of mice treated with PBS or no treatment (healthy) without receiving DSS is shown as a control (p values relative to mice receiving DSS and PBS are indicated); and

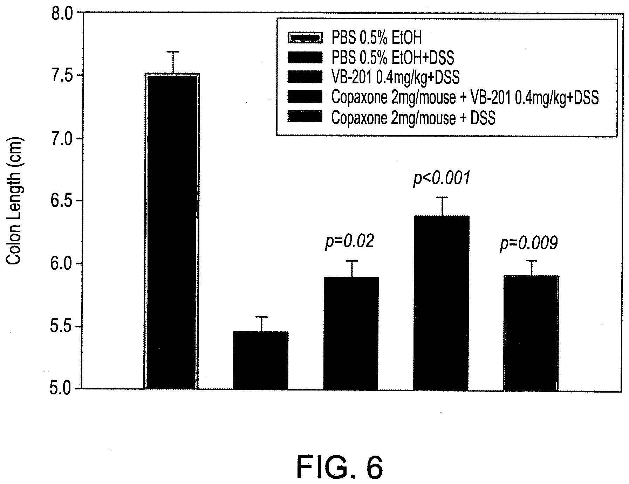

FIG. 6 is graph showing the colon length of mice treated with 0.4 mg/kg VB-201 and/or 2 mg per mouse glatiramer acetate or PBS (with 0.5 % ethanol) 39 days following induction of colitis by DSS; colon length of mice treated with PBS or no treatment (healthy) without receiving DSS is shown as a control (p values relative to mice receiving DSS and PBS are indicated).

## DESCRIPTION OF SPECIFIC EMBODIMENTS OF THE INVENTION

The present invention, in some embodiments thereof, relates to the field of pharmacology and more particularly to novel dosages, treatment regimens and therapeutic uses of the oxidized phospholipid VB-201.

The principles and operation of the present invention may be better understood with reference to the figures and accompanying descriptions.

VB-201 (also referred to herein and in the art as CI-201) has shown considerable promise as a therapeutically active agent in various *in vitro* models and *in vivo* animal models of inflammatory conditions.

In an attempt to improve treatment of inflammatory diseases and disorders, the present inventors have studied in detail the effects and mechanism of action of VB-201 when administered in combination with additional anti-inflammatory agents in *in vivo* models. The protocols of these assays are described in detail in the Examples section that follows. Based on the data obtained in the studies conducted, the present inventors have developed improved treatment regimens and methodologies.

Referring now to the drawings and tables, Table 1 shows that atorvastatin reduces cholesterol levels, whereas VB-201 has no effect on cholesterol levels, in contrast to the therapeutic affect of atorvastatin. Figure 1 shows that although VB-201 clearly did not reduce cholesterol levels, VB-201 surprisingly and synergistically enhances the ability of statins to inhibit lesion growth. Table 2 shows that atorvastatin increases levels of the liver enzymes SGPT and alkaline phosphatase, whereas VB-201 does not increase levels of the liver enzymes, and may even attenuate atorvastatin-induced increases of alkaline phosphatase levels.

Figures 2 and 3 show that glatiramer acetate was particularly effective immediately after onset of administration in a mouse colitis model, whereas VB-201 was particularly effective in the long run. These results indicate that glatiramer acetate and VB-201 have different therapeutic effects which can complement one another synergistically.

Figures 4 and 5 show that VB-201 and glatiramer acetate each partially reverse inflammatory effects in a mouse colitis model. Figure 6 shows that co-administration of VB-201 and glatiramer acetate reverses inflammatory effects to a degree greater than the sum of the effects of administration of VB-201 and glatiramer acetate alone. Figures 4 and 5 further show that the therapeutic effect of VB-201 is not dose-dependent at the administered doses, and therefore cannot be enhanced by administration of higher doses. This further confirms that the enhanced therapeutic effect observed with co-administration of VB-201 and glatiramer acetate is not merely an additive effect.

Thus, the results presented herein demonstrate that VB-201 can advantageously enhance the therapeutic effects of statins and other lipid-lowering drugs (e.g., fibrates, niacin, bile acid sequesterants, eztimibe, phytosterols, orlistat, CETP inhibitors, squalene synthase inhibitors, and other drugs for reducing cholesterol levels as is further detailed hereinbelow) in a surprising and synergistic manner, without a corresponding enhancement of adverse side effects, and can surprisingly even reduce adverse side effects caused by statins and/or other lipid-lowering drugs. Thus, VB-201 has a potentiating and a sparing effect on the dosage of statin (i.e., reduces the necessary dosage) needed for effective treatment (e.g., of cardiovascular disease). Based upon these findings, the present inventors have devised improved regimens which utilize the sparing effect of VB-201 in the context of statin administration (the statin-sparing effect of VB-201) and/or administration of other lipid-loweing drugs.

Hence, there is described

a method of treating a cardiovascular disease. The method comprises administering a therapeutically effective amount of a statin to a subject in need thereof, determining a responsiveness of the subject to the therapeutically effective amount of the statin, to thereby determine if the subject is not fully responsive to the therapeutically effective amount, and, if the subject is determined as not fully responsive to the therapeutically effective amount of the statin, administering to the subject a therapeutically effective amount of VB-201.

A subject may be administered a statin based on the instructions of a physician, which may be based on any combination of various factors such as high plasma LDL cholesterol levels and/or triglyceride levels, low HDL cholesterol levels, high blood pressure, and/or a family history of cardiovascular disease.

Herein, the phrase "therapeutically effective amount" denotes a dosage of an active ingredient (e.g., statin) that is expected, based on e.g., clinical studies and practice, by one of skill in the art (e.g., a physician) to provide, in at least a portion of subjects receiving such a dosage, a therapeutic effect for which the active ingredient is indicated, for example, reducing a cholesterol level (e.g., when the active ingredient is a statin).

It is to be appreciated that a therapeutically effective amount is not necessarily expected to provide a full response, as this term is used herein. It is to be further appreciated that because different subjects often respond in different manners to therapeutic agents, a commonly used therapeutically effective amount may, in some subjects, not provide any therapeutic effect whatsoever.

Optionally, the method comprises evaluating a response of the subject to the administered amount of statin, in order to then determine if the subject is fully responsive.

Evaluation of the response may comprise any medical evaluation (e.g., physical examination, biochemical tests) for evaluating the cardiovascular disease (e.g. evaluating the presence and/or progression of the disease) and/or a statin activity (including therapeutic and non-therapeutic statin activities).

Herein, the term "responsive" refers to a therapeutically beneficial effect in the subject in response to administration of the statin. A subject is considered "fully responsive" if the response achieves a desired effect which is known in the art to be obtainable by administration of the statin. A subject who is not fully responsive may be a subject in which no substantial response was observed (e.g., administration of the statin had no therapeutic effect) or a subject in which a response was observed, but the desired effect was not achieved (e.g., the therapeutic effect was too small).

The evaluation of responsiveness may comprise a blood test.
Examples of biochemical markers which may be utilized for determining responsiveness to administration of a statin include, without limitation, hs-CRP levels, low-density lipoprotein (LDL) cholesterol levels, Lp-PLA$_2$ (lipoprotein-associated phospholipase A$_2$) levels, apolipoprotein B100 (APOB100) levels, apolipoprotein A (APOA) levels, triglyceride levels, lipoprotein(a) levels, high-density lipoprotein (HDL) cholesterol levels, and total cholesterol levels.

The term "hs-CRP" refers to a high sensitivity assay for C-reactive protein (CRP), which is well known in the art. This assay is the preferred method for determining CRP levels in a subject, as the high sensitivity allows for detection of low blood levels and small differences in CRP levels. The phrase "hs-CRP level" refers herein to a level of CRP, as determined by hs-CRP assay, or any equivalent technique used by a skilled practitioner.

In some embodiments, a subject is considered not fully responsive when there is no reduction of cholesterol, as determined, for example, by measurement of total cholesterol and/or LDL (e.g., as described herein).

A subject may be considered not fully responsive when cholesterol is reduced, but there is no reduction of hs-CRP levels (as described herein).

A subject may be considered not fully responsive when cholesterol and hs-CRP levels are reduced (e.g., as described herein), but the subject exhibits signs of cardiovascular disease progression.

Optionally, full responsiveness is determined quantitatively, wherein one or more parameters are above (or below) a selected cutoff level. Alternatively or additionally, full responsiveness is determined quantitatively, wherein a change in one or more parameters is of at least a selected degree, for example a reduction (or increase) of at least a selected percentage (e.g., 50 %).

For example, statins are commonly used in order to lower plasma cholesterol levels, particularly low density lipoprotein (LDL) cholesterol levels, although total cholesterol levels are also frequently used as a marker.

Thus, the desired effect in the context of administration of statins is optionally a reduction of plasma cholesterol levels (e.g., LDL cholesterol, total cholesterol). Optionally, a degree of responsiveness is determined by an extent of a reduction of plasma cholesterol levels (e.g., LDL cholesterol, total cholesterol).

Thus, a subject not fully-responsive to a statin treatment is optionally a subject that has an elevated plasma cholesterol level (e.g., a level above a desired end point for the subject), optionally an elevated plasma LDL cholesterol level, and in which the statin treatment did not reduce the plasma cholesterol level, or reduced the plasma cholesterol level but not to the desired end point.

The desired end point for the subject may optionally be selected according to the apparent degree of risk for cardiovascular events in the subject, as assessed by a skilled practitioner according to criteria used in the art. The degree of risk may be determined, for example, based on whether the subject has previously had a heart attack, stroke and/or aortic aneurysm, as well as the presence of recognized risk factors (e.g., high blood pressure, metabolic syndrome, poor diet, sedentary lifestyle, old age, smoking, a family history of cardiovascular disease, pregnancy, menopause, diabetes, and certain thyroid conditions).

A desired end point in a subject may optionally change over time, for example, if there is a change in the apparent risk of the subject for cardiovascular events. A desired end point may be changed, for example, based on instructions of a physician treating the subject.

In addition, the efficacy of a statin in a subject may be determined by monitoring a marker for cardiovascular disease, such as serum hs-CRP levels.

Thus, the desired effect is optionally a reduction of serum CRP levels, and optionally a reduction of both serum LDL cholesterol and serum CRP levels. Optionally, a degree of responsiveness is determined by an extent of a reduction of serum CRP levels.

A subject who is not fully responsive may be characterized by serum CRP levels of at least a selected cutoff level. Optionally, the cutoff level is 1.1 mg/L. Optionally, the cutoff level is 2 mg/L.

A subject who is not fully responsive may be characterized by plasma LDL cholesterol levels (e.g., fasting levels) of at least a selected cutoff level.

The cutoff level may optionally be selected according to the individual risk of the subject for cardiovascular disease (e.g., according to criteria commonly used by physicians).

Thus, for example, in high risk subjects, the LDL cutoff level is optionally about 70 mg/dL. Examples of high risk subjects include subjects who have already had a heart attack or stroke, as well as an additional condition such as metabolic syndrome, high blood pressure and/or smoking.

In subjects diagnosed with diabetes, the LDL cutoff level is optionally in a range of about 70 mg/dL to about 80 mg/dL (e.g., 70 mg/dL, 80 mg/dL).

In subjects who have already had a vascular event, or who have proven atherosclerosis, coronary artery disease (CAD) or another vascular disease, such as carotid artery disease, peripheral artery disease, or a previous abdominal aortic aneurysm, the LDL cutoff level is optionally about 100 mg/dL.

For lower risk subjects, the cutoff level may optionally be, for example in a range of about 100-190 mg/dL. Optionally, the cutoff level is in a range of about 100-130 mg/dL (e.g., about 100 mg/dL, about 130 mg/dL) for a subject having two or more risk factors described herein. Optionally, the cutoff level is in a range of about 130-190 mg/dL (e.g., about 130 mg/dL, about 160 mg/dL, about 190 mg/dL) for a subject having no more than one such risk factor.

A subject who is not fully responsive may be characterized by plasma LDL levels which do not decrease upon statin administration, or which decrease by less than a selected percentage (e.g., 20 %, 30 %, 40%, 50 %). The selected percentage may be selected according to the individual risk of the subject for cardiovascular disease. Thus, in high risk subjects (e.g., as described herein), the selected percentage is optionally 50 %, and for diabetic subjects, the selected percentage is optionally about 35 %. For lower risk subjects, the percentage is optionally lower.

A subject who is not fully responsive may be characterized by either plasma LDL cholesterol levels (e.g., fasting levels) of at least a selected cutoff level (e.g., as described herein) or plasma LDL levels which do not decrease by at least a selected percentage (e.g., 50 %).

For example, in high risk subjects (e.g., as described herein), a subject is optionally considered not fully responsive unless plasma LDL levels decrease by at least 50 %, and to a level below 70 mg/dL.

In another example, a diabetic subject is optionally considered not fully responsive unless plasma LDL levels decrease by at least 35 %, and to a level below 70-80 mg/dL.

LDL levels may be evaluated by any method commonly used in the art, including direct measurement, and from measurement of other parameters, as is commonly performed in the art. For example, LDL cholesterol concentration may be estimated by the formula:

$$[\text{LDL cholesterol}] = [\text{total cholesterol}] - [\text{HDL cholesterol}] - (0.2 \times [\text{triglycerides}])$$

A subject who is not fully responsive may be characterized by Lp-PLA$_2$ levels of at least a selected cutoff level. Optionally, the cutoff level is at least about 200 mg/dL (e.g., about 200 mg/dL, about 235 mg/dL).

A subject who is not fully responsive may be characterized by apolipoprotein B100 levels of at least a selected cutoff level. Optionally, the cutoff level is at least about 125 mg/dL (e.g., 125 mg/dL).

A subject who is not fully responsive may be characterized by apolipoprotein A levels below a selected cutoff level. Optionally, the cutoff level is about 100 mg/dL or less (e.g., 100 mg/dL).

A subject who is not fully responsive may be characterized by blood triglyceride levels of at least a selected cutoff level. Optionally, the cutoff level is at least about 150 mg/dL (e.g., 150 mg/dL).

A subject who is not fully responsive may be characterized by lipoprotein(a) levels of at least a selected cutoff level. Optionally, the cutoff level is at least about 14 mg/dL (e.g., 14 mg/dL), and optionally at least about 30 mg/dL (e.g., about 30 mg/dL, about 50 mg/dL).

A subject who is not fully responsive may be characterized by high-density lipoprotein (HDL) cholesterol levels below a selected cutoff level. Optionally, the cutoff level is about 60 mg/dL or less, and optionally 55 mg/dL or less in female subjects (e.g., about 55 mg/dL, about 50 mg/dL), and optionally 45 mg/dL or less in female subjects (e.g., about 45 mg/dL, about 40 mg/dL).

A subject who is not fully responsive may be characterized by total plasma cholesterol levels of at least a selected cutoff level. Optionally, the cutoff level is at least 200 mg/dL (e.g., 200 mg/dL).

The cutoff levels described herein may optionally be selected according to the individual risk of the subject for cardiovascular disease.

When a subject is considered not fully responsive based upon more than one of any of the criteria described herein, the subject is optionally considered not fully responsive whenever at least one criterion is met. Alternatively, the subject is considered not fully responsive only when each criterion is met.

Thus, VB-201 is specifically administered to a subject particularly likely to benefit from VB-201, e.g., a subject who is not fully responsive to administration of the statin alone.

The administration of a statin described herein may comprise any dosage of the statin.

The method may further comprises determining if a subject is vulnerable to an adverse effect of a dosage of statin higher than the therapeutically effective amount administered as described herein. Optionally the therapeutically effective amount of VB-201 described herein is administered to the subject who is both not fully responsive to the administered amount of statin, and who is vulnerable to the adverse effect of a dosage of statin higher than the administered therapeutically effective amount described herein.

Thus, VB-201 is administered to a subject who is particularly likely to benefit from VB-201 (e.g., a subject who is not fully responsive to administration of the statin alone) and who cannot be effectively treated by raising the dosage of statin, without creating a severe risk of an adverse effect.

Determination whether the subject is vulnerable to an adverse effect may be by administering a dosage of statin to the subject and identifying an adverse effect in the subject, thereby determining that the subject is vulnerable to an adverse effect of such a dosage of statin. The dosage of statin administered to the subject may then be reduced (e.g., by at least 10 %, 20 %, 30 %, 40 %, 50 %) the therapeutically effective amount described herein.

Optionally, the dosage is reduced until reaching a level for which no adverse effect is observed.

Alternatively, the dosage is reduced to a level for which a mild adverse effect is observed.

Examples of relevant adverse events which may be caused by statins include, without limitation, elevated levels in the blood of one or more liver enzymes, myalgia, muscle cramps, polyneuropathy, myositis, myopathy, rhabdomyolysis and acute renal failure.

It is to be appreciated that some adverse events (e.g., relatively non-harmful adverse events) may optionally be considered only upon reaching a certain degree of severity. For example, mild muscle cramps (e.g., muscle cramps which do not significantly reduce quality-of-life) and moderate increases in liver enzyme levels (e.g., increases which do not indicate significant harm to the subject's health) may optionally not be considered adverse events, whereas severe muscle cramps and dramatic increases in liver enzyme would be considered as adverse events. It is within the capabilities of one skilled in the art to determine when an adverse effect is severe (e.g., indicating a significant risk to the subject's health and/or quality-of-life).

The subject may be determined to be vulnerable to an adverse effect of a dosage of statin higher than the administered therapeutically effective amount, when the administered amount is near or at a maximum acceptable dosage of the statin (e.g., in a range of from 50 % to 100 % of maximum acceptable dosage described herein).

The statin may be administered at a dosage which is escalated when the subject is determined to be not fully responsive to the dosage, such that if the subject is not fully responsive to any of the escalating dosages, eventually a point is reached for which an additional escalation is considered undesirable, for example, if additional escalation would result in a dosage above a maximum acceptable dosage.

Herein a "maximum acceptable dosage" refers to the highest dosage recognized as being acceptable for administration, for example, the highest dosage for which adverse events are not expected or are expected to be at a level which is acceptable for treatment of a subject. The maximum acceptable level may optionally depend on an age, sex, medical condition (e.g., the severity of the disease being treated, the severity of the adverse side effect and/or the general health condition of the subject and the effect of an optional side effect thereon), genetic profile, and/or ethnicity of the subject.

The maximum acceptable level may optionally be determined based on the reported literature (e.g., medical textbooks, medical journals), regulations (e.g., hospital regulations, government regulations) and/or advice of a recognized organization or agency.

Optionally, the maximum acceptable dosage is a maximum recommended dosage of a recognized organization or agency (e.g., the U.S. Food and Drug Administration (FDA) and/or the European Medicines Agency).

When such an organization or agency recommends a range of dosages, the "maximum recommended dosage" refers to the highest dosage in the range, and when a single dosage is recommended, the "maximum recommended dosage" refers to the recommended dosage.

As used herein, a "statin" encompasses any compound recognized in the art as a statin and/or a HMG-CoA reductase inhibitor, and any combination of such compounds. Examples of currently marketed and/or regulatorily approved statins include, without limitation, atorvastatin, fluvastatin, lovastatin, pitavastatin, pravastatin, rosuvaststin, and simvastatin. Additional known statins include cerivastatin and mevastatin.

It is expected that during the life of a patent maturing from this application many relevant statins will be developed and the scope of the term "statin" is intended to include all such new compounds *a priori*.

The maximum acceptable dosage of the statin may be 80 mg/day (e.g., 80 mg/day of atorvastatin, fluvastatin, lovastatin, or simvastatin).

The maximum acceptable dosage may be 40 mg/day of pravastatin or rosuvastatin.

Optionally, the "therapeutically effective amount of a statin" described herein is administered in combination with one or more additional agents, such that a subject who is not fully responsive to the statin, as described herein (e.g., as determined by a failure to reduce cholesterol levels), is not fully responsive to the combined treatment of statin and additional agent(s).

Examples of agents which may be administered in combination with the statin include, without limitation, a mucosal adjuvant, a corticosteroid, a steroidal anti-inflammatory drug, a non-steroidal anti-inflammatory drug, an analgesic, a growth factor, a toxin, a heat-shock protein, a beta-2-glycoprotein I, a cholesteryl ester transfer protein (CETP) inhibitor, a peroxisome proliferative activated receptor (PPAR) agonist, an anti-atherosclerosis drug, an anti-proliferative agent, nicotinic acid, a bile acid sequestrant, a cholesterol absorption inhibitor (including triglyceride absorption inhibitors), a cholesterol biosynthesis inhibitor (e.g., a squalene inhibitor), ApoE Milano, an angiotensin-converting enzyme inhibitor (such as captopril, enalapril, or lisinopril), an antiarrhythmic drug (such as amiodarone), an anticoagulants, an antiplatelet or thrombolytic agent (such as aspirin), a centrally acting antihypertensive (such as clonidine, guanfacine or methyldopa), a digitalis drug (such as digoxin), a diuretic (such as chlorthalidone), a nitrate (such as nitroglycerin), a peripheral adrenergic antagonist (such as reserpine), and a vasodilators (such as hydralazine).

Optionally, the additional agent(s) is for reducing cholesterol levels.

PPAR agonists, also known as fibrates, are fatty acid-activated members of the nuclear receptor superfamily that play important roles in lipid and glucose metabolism, and have been implicated in obesity-related metabolic diseases such as hyperlipidemia, insulin resistance, and coronary artery disease. Fibrates are generally effective in lowering elevated plasma triglycerides and cholesterol and act as PPAR agonists. The most pronounced effect of fibrates includes a decrease in plasma triglyceride-rich lipoproteins (TRLs). Levels of LDL cholesterol generally decrease in individuals with elevated baseline plasma concentrations, and HDL cholesterol levels are usually increased when baseline plasma concentrations are low. Non-limiting examples of commonly prescribed fibrates include bezafibrate, gemfibrozil and fenofibrate.

Representative examples of cholesterol absorption inhibitors include ezetimibe. Ezetimibe is the first of a new class of cholesterol absorption inhibitors that potently and selectively inhibits dietary and biliary cholesterol absorption at the brush border of the intestinal epithelium, without affecting the absorption of triglyceride or fat-soluble vitamins. Ezetimibe thus reduces overall cholesterol delivery to the liver, secondarily inducing increased expression of LDL receptors, resulting in an increased removal of LDL cholesterol from the plasma.

Cholesterol absorption may also be affected by cholesteryl ester transfer protein (CETP) inhibitors, which play a major role in atherogenesis, by reducing cholesteryl ester accumulation within macrophages and the arterial wall, and thus reducing foam cell formation and affecting the cholesterol absorption. The most promising presently known CETP inhibitor is anacetrapib.

Bile acid sequestrants are a class of cholesterol lowering drugs that help rid the body of cholesterol by depleting cholesterol levels in the body. Bile is released from the liver and aids in the emulsification of fats. Cholesterol is a major component of bile, and most of the cholesterol from bile is reabsorbed into the bloodstream in the small intestine. Bile acid sequestrants act at the level of the small intestine and function in binding to bile, thus preventing cholesterol from being reabsorbed into circulation. Instead, the medication and bile will form an insoluble complex and be excreted in the feces. Examples of commonly prescribed bile acid sequesterants include cholestyramine (e.g., Questran®), colesevelam, and colestipol (e.g., Colestid®).

Representative examples of cholesterol biosynthesis inhibitors include squalene inhibitors (such as squalene monooxygenase inhibitors and squalene synthase inhibitors). Squalene is an isoprenoid compound structurally similar to beta-carotene, is an intermediate metabolite in the synthesis of cholesterol. In humans, about 60 percent of dietary squalene is absorbed. It is transported in serum generally in association with very low density lipoproteins and is distributed ubiquitously in human tissues, with the greatest concentration in the skin, where it is one of the major components of skin surface lipids. Squalene inhibitors (e.g., squalene monooxygenase and squalene synthase inhibitors) serve as cholesterol biosynthesis inhibitors.

Nicotinic acid (or niacin), a water-soluble B vitamin, is a known agent that lowers total cholesterol, LDL-cholesterol, and triglyceride levels, while raising HDL-cholesterol levels. There are three types of nicotinic acid drugs:

immediate release, timed release, and extended release. Nicotinic acid improves all lipoproteins when given in doses well above the vitamin requirement.

The regimens described hereinabove are designed suitable for achieving an effective co-administration of statin and VB-201 in individual subjects.

The cardiovascular disease which may be treated is optionally an inflammatory cardiovascular disease or disorder. Suitable inflammatory cardiovascular disease or disorders include, without limitation, occlusive diseases or disorders, atherosclerosis, a cardiac valvular disease, stenosis, restenosis, in-stent-stenosis, myocardial infarction, coronary arterial disease, acute coronary syndromes, congestive heart failure, angina pectoris, myocardial ischemia, thrombosis, Wegener's granulomatosis, Takayasu's arteritis, Kawasaki syndrome, anti-factor VIII autoimmune disease or disorder, necrotizing small vessel vasculitis, microscopic polyangiitis, Churg and Strauss syndrome, pauci-immune focal necrotizing glomerulonephritis, crescentic glomerulonephritis, antiphospholipid syndrome, antibody induced heart failure, thrombocytopenic purpura, autoimmune hemolytic anemia, cardiac autoimmunity, Chagas' disease or disorder, and anti-helper T lymphocyte autoimmunity.

Stenosis is an occlusive disease of the vasculature, commonly caused by atheromatous plaque and enhanced platelet activity, most critically affecting the coronary vasculature.

Restenosis is the progressive re-occlusion often following reduction of occlusions in stenotic vasculature. In cases where patency of the vasculature requires the mechanical support of a stent, in-stent-stenosis may occur, re-occluding the treated vessel.

The monitoring of a responsiveness of a subject to a regimen may also be used to determine a therapeutically effective amount of VB-201 for a particular individual. Determining such a therapeutically effective amount is beneficial, as the dosage which is most therapeutically effective is likely to differ between individuals. Consequently, designing regimens suitable for each individual can result in stronger therapeutic effects and/or less adverse side effects.

Hence, there is described a method of determining a therapeutically effective amount of VB-201 for administration in a subject. The method comprises administering to the subject a dosage of VB-201, determining a responsiveness of the subject to the dosage of VB-201, to thereby determine if the subject is not fully responsive to the dosage of VB-201, and escalating the dosage of VB-201 (if the subject was not fully responsive to the previous dosage) administered to the subject until the subject is fully responsive to the dosage and/or until a maximal dosage is reached.

The therapeutically effective amount of VB-201 to be determined is optionally a therapeutically effective amount for the treatment of an inflammatory disease or disorder. The responsiveness thus optionally comprises alleviating a symptom of such a disease or disorder.

The escalation of a dosage described optionally comprises increasing the dosage by 25 % to 300 % for each escalation of the dosage, optionally by 50 % to 100 %, and optionally by about 100 %.

Optionally, the method further comprises monitoring adverse effects which occur in the subject being administered VB-201.

The maximal dosage may be the highest tolerated dosage of VB-201 in the subject.

As used herein, the term "tolerated" refers to the absence of adverse effects in the subject or to the presence of tolerable adverse effects (e.g., mild side effects). Optionally, mild adverse effects (e.g., as described herein) are not considered for the purposes of determining whether a dosage is tolerated.

A highest tolerated dosage of VB-201 may optionally be determined by administering a higher dosage of VB-201 to the subject and identifying an adverse effect in the subject, and then reducing the dosage to the highest tolerated dosage.

A highest tolerated dosage may also optionally be determined based on a maximum acceptable dosage of VB-201 (e.g., an FDA and/or European Medicines Agency maximum acceptable dosage). Optionally, the highest tolerated dosage is in a range of from 50 % to 200 %, optionally from 75 % to 150 %, and optionally about 100 % of a maximum acceptable dosage.

The maximal dosage may be a dosage for which the responsiveness of the subject is at least as high as a responsiveness of the subject to a higher dosage. For example, a saturation effect may be observed, wherein escalating the dosage beyond a certain amount does not increase the responsiveness of the subject. In cases wherein a range of dosages provide approximately the same effect (e.g., responsiveness) in a subject, it is generally desirable to use a low dosage (e.g., the lowest dosage) within that range, for example, in order to minimize the likelihood and/or severity of possible adverse effects.

The maximal dosage may be either:

a) the highest tolerated dosage (as described herein); and/or
b) a dosage for which the responsiveness of the subject is at least as high as a responsiveness to a higher dosage (as described herein),

whichever dosage is lower.

Responsiveness is optionally determined by determining a presence and/or level of a biomarker for a condition in the subject (e.g., by blood test), for example, a biomarker for inflammation. An elevated hs-CRP level (e.g., 1.1 mg/L or higher) may optionally be used as a biomarker for inflammation, as described herein.

The VB-201 is administered alone. Optionally, the therapeutically effective amount determined may be a therapeutically effective amount of VB-201 for administration of VB-201 alone.

The VB-201 may be administered in combination with an additional therapeutically active agent. Optionally, the therapeutically effective amount determined may be a therapeutically effective amount of VB-201 for administration in combination with the additional therapeutically active agent. The additional therapeutically active agent may comprise a statin and/or any other agent described herein as suitable for administration in combination with VB-201.

It is to be understood that the phrase "additional therapeutically active agent" is intended to encompass a combination of a plurality of therapeutically active agents.

As exemplified in the Examples section below, the present inventors have further devised methods for achieving a general regimen comprising co-administration of VB-201 and an additional therapeutic agent (e.g., a statin) which is designed suitable for a general population of subjects.

Hence, there is described a method of determining a therapeutically effective amount of VB-201 for administration in combination with an additional therapeutically active agent. The method comprises administering different dosages of VB-201 to subjects, each subject receiving a particular dosage, with a portion of the subjects being administered a placebo instead of VB-201. The subjects are selected as being treated with a therapeutically effective amount of the additional therapeutically active agent (also referred to herein simply as the "therapeutically active agent"), and being not fully responsive (as defined herein) to the therapeutically effective amount of the therapeutically active agent.

The method further comprises monitoring a responsiveness of the subjects (e.g., as described herein) to the administered dosages of VB-201 in combination with the administered therapeutically effective amount of the therapeutically active agent, as well as adverse effects which occur in the subjects, and identifying at least one dosage for which the subjects are responsive.

The subjects may be monitored as groups, wherein each group comprises a plurality of subjects being administered the same dosage of VB-201 and/or the therapeutically active agent, or dosages of VB-201 and/or the therapeutically active agent which are each within a selected range. Such grouping of subjects allows for statistical analysis (e.g., obtaining means and standard deviation from the mean) of the results of the monitoring. Optionally each group comprises at least 5 subjects, optionally at least 10 subjects, and optionally at least 20 subjects.

Optionally, the subjects are administered a relatively high dosage of the therapeutically active agent, for example, at least 25 %, optionally at least 50 %, and optionally at least 75 %, of a maximum acceptable dosage (as defined herein) of the therapeutically active agent.

The therapeutically active agent is optionally selected so as to be suitable for treating an inflammatory disease or disorder (e.g., as described herein). Optionally, the therapeutically active agent is for treating a cardiovascular disease, for example, a cardiovascular disease described herein.

The therapeutically active agent may be a highly potent statin (e.g., a statin described herein). Exemplary dosages of statin include at least 20 mg/day atorvastatin, at least 10 mg/day rosuvastatin, and at least 40 mg/day simvastatin.

When the therapeutically active agent (e.g., a statin) is for treating inflammation (e.g., an inflammatory cardiovascular disease), responsiveness of a subject to the therapeutically active agent (e.g., in order to identify a subject not fully responsive to the therapeutically effective amount of the agent, as described herein) and/or to the co-administration of the therapeutically active agent and VB-201 (e.g., in order identify a dosage of VB-201 for which the subjects are responsive, as described herein) may optionally be characterized by measuring an inflammatory biomarker, for example, hs-CRP, IL-1$\beta$, IL-6, IL-12, IL-17, IL-22, IL-23, IFN-$\alpha$, IFN-$\gamma$, TNF-$\alpha$, MCP-1, MIP-1$\alpha$, MIP-1$\beta$, IL-12 p40, IL-12 p70, MPO, SAA and/or IL-8 (e.g., as described herein). Optionally, responsiveness is characterized by a reduction in the inflammatory biomarker to a desired end point, as determined by a physician.

Responsiveness may be characterized by CRP levels below a cutoff level described herein (e.g., 1.1 mg/L) and/or any significant (e.g., at least 10 %, at least 20 %, at least 30 %, at least 50 %) reduction of CRP levels from the baseline levels.

The therapeutically active agent (e.g., a statin) may be for treating an inflammatory cardiovascular disease, and responsiveness of a subject to the therapeutically active agent (e.g., in order to identify a subject not fully responsive to the therapeutically effective amount of the agent, as described herein), but not to the co-administration of the therapeutically active agent and VB-201, is characterized by measuring LDL, Lp-PLA$_2$, APOB100, APOA, triglycerides, lipoprotein(a), HDL and/or total cholesterol levels, as described herein.

Responsiveness may be characterized by an absence of progression of a disease being treated (e.g., a cardiovascular disease).

As discussed hereinabove, and exemplified in the Examples section below, VB-201 exhibits a synergistic anti-inflammatory effect when co-administered with glatiramer acetate.

Hence, there is described a method of treating an inflammatory disease or disorder, the method comprising co-administering a therapeutically effective amount of VB-201 and a therapeutically effective amount of glatiramer acetate.

Optionally, the therapeutically effective amount of glatiramer acetate is in a range of from 2-200 mg/day, optionally 4-100 mg/day, optionally 10-50 mg/day, and optionally about 20 mg/day.

The glatiramer acetate is administered according to techniques used in the art for administration of glatiramer acetate (e.g., injection).

The term "glatiramer acetate" is intended to encompass all structurally related copolymers of glutamic acid, lysine, alanine and tyrosine, and should not be interpreted as being limited to acetate salts thereof.

As described herein, glatiramer acetate provides an effective anti-inflammatory effect shortly after the onset of administration (during which time VB-201 may not exhibit a strong effect), but may provide little or any benefit later on.

Hence, the method may further comprise ceasing administration of glatiramer acetate (e.g., when glatiramer acetate is not expected to continue providing a significant therapeutic benefit) and continuing to administer a therapeutically effective amount of VB-201, which is expected to provide a therapeutic effect over a long period of time.

Optionally the co-administration is effected for a period of time in a range of at least 1 week, and optionally at least 2 weeks. Optionally co-administration is for up to 1 year, optionally up to 6 months, optionally up to 3 months, and optionally for 1 month or less.

The continued administration of VB-201 alone (i.e., after administration of glatiramer acetate has ceased) may be for any time period (e.g., for life), as needed.

Co-administration of glatiramer acetate and VB-201 may be for any time period (e.g., for life), as needed.

As discussed herein, and exemplified in the Examples section, a therapeutically effective amount of VB-201 for administration may be determined to be a dosage of over 100 mg per day (e.g., 120 mg per day, 160 mg per day, 240 mg per day).

Hence, there is described a method of treating or preventing an inflammatory disease or disorder, the method comprising orally administering to a subject in need thereof a therapeutically effective amount of at least of VB-201, wherein the therapeutically effective amount is more than 100 mg per day, optionally from 101 mg to 1 gram per day, and optionally from 101 mg to 300 mg per day.

The therapeutically effective amount may be in a range of from 101 mg to 140 mg per day, and optionally about 120 mg per day.

The therapeutically effective amount may be in a range of from 120 mg to 200 mg per day, and optionally about 160 mg per day. Optionally, a method utilizing such a therapeutically effective amount is for treating an inflammatory disease or disorder such as rheumatoid arthritis.

The therapeutically effective amount may be in a range of from 200 mg to 280 mg per day, and optionally about 240 mg per day.

The therapeutically effective amounts described herein refer to therapeutically effective amounts for administration to adult subjects. Optionally, amounts of VB-201 to be administered to a child are adjusted, for example, according to body weight of the child.

The therapeutically effective amounts described herein may be absolute, and do not depend on the body weight of the subject, with the optional exception of subjects with a body weight far from the adult average (e.g., children).

The therapeutically effective amounts described herein may be considered suitable for a subject of average body weight (e.g., 70 kg), and the amount to be administered is adjusted according to the body weight of the subject receiving VB-201. For example, a dosage of 240 mg per day recited herein is optionally understood to refer to a dosage of 240 mg per 70 kg per day body weight (i.e., 3.43 mg/kg).

The therapeutically effective amount administered daily may be divided into a plurality (e.g., 2 or 3) of administrations, for example, at pre-determined intervals.

The administration of the therapeutically effective amount may be effected by administering VB-201 once per day. Administration of VB-201 once per day typically results in relatively stable VB-201 plasma concentrations, which are often desirable during treatment.

The administration of the therapeutically effective amount may be effected by administering VB-201 at least twice per day, optionally twice per day, which may result in even more stable plasma concentrations.

The therapeutically effective amount of VB-201 may be formulated as unit dosage forms of a medicament (e.g., a pharmaceutical composition) designed for easy and convenient administration of a therapeutically effective amount of VB-201 described herein.

Hence, there is described a pharmaceutical composition unit dosage form comprising more than 100 mg VB-201 (optionally from 101 mg to 1 gram, and optionally from 101 mg to 300 mg) and a pharmaceutically acceptable carrier, the pharmaceutical composition unit dosage form being formulated for oral administration.

The therapeutically effective amount may be in a range of from 101 mg to 140 mg, and optionally about 120 mg.

The therapeutically effective amount may be in a range of from 200 mg to 280 mg, and optionally about 240 mg.

The administration of more than 100 mg per day VB-201 according to the method described herein, may be conveniently effected by administration of one or two of the unit dosage forms described herein per day. For example, a unit dosage comprising about 120 mg VB-201 is suitable for administering about 120 mg VB-201 per day (when one unit dosage form is administered per day) or about 240 mg VB-201 per day (when two unit dosage forms are administered per day).

The method described herein may also be effected using unit dosage forms comprising less than 101 mg VB-201, for example, when VB-201 is administered twice or more per day. Thus, for example, administration of 160 mg VB-201 per day may be effected by administering two dosage forms (e.g., at different times during the day) comprising 80 mg VB-201, administration of 120 mg VB-201 per day may be effected by administering two dosage forms comprising 60 mg VB-201 or by administering a dosage form comprising 80 mg VB-201 and a dosage form comprising 40 mg VB-201, and so forth.

Various dosage forms of less than 100 mg VB-201 are described, for example, in U.S. Provisional Patent Application Nos. 61/292,226 and/or 61/282,250 and in PCT International Patent Application entitled "TREATMENT WITH VB-201", having attorney's Docket No. 50377, which is co-filed with the instant application.

Thus, for example, the daily dosage may be effected by administering a plurality of unit dosage forms comprising, for example, 10 mg, 20 mg, 30 mg, 40 mg, 50 mg, 60 mg, 70 mg, 80 mg, 90 mg and/or 100 mg VB-201. Any combination of such unit dosage forms which results in a daily dosage higher than 100 mg by multiple daily administrations is disclosed

The pharmaceutical unit dosage forms described herein are optionally packaged in a packaging material and identified in print, in or on the packaging material, for use in the treatment or prevention of an inflammatory disease or disorder (e.g., a disease or disorder described herein). A plurality of unit dosage forms may be packaged in a packaging material and identified in print, in or on the packaging material, for use in the treatment or prevention of an inflammatory disease or disorder.

There is described a use of VB-201 in the manufacture of a unit dosage form of a medicament for treating or preventing an inflammatory disease or disorder (e.g., a disease or disorder described herein), the unit dosage form comprising more than 100 mg VB-201 (e.g., as described herein), and being formulated for oral administration

As used herein, a "pharmaceutical composition" refers to a preparation of VB-201 (as active ingredient), or physiologically acceptable salts thereof, with other chemical components, including, but not limited to, physiologically suitable carriers, excipients, lubricants, buffering agents, antibacterial agents, bulking agents (e.g. mannitol), antioxidants (e.g., ascorbic acid or sodium bisulfite), and the like. The purpose of the pharmaceutical composition is to facilitate administration of VB-201 to a subject.

The term "unit dosage form", as used herein, describes physically discrete units, each unit containing a pre-determined quantity of VB-201 calculated to produce the desired therapeutic effect, in association with at least one pharmaceutically acceptable carrier, diluent, excipient, or combination thereof.

Herein, the phrases "physiologically acceptable carrier" and "pharmaceutically acceptable carrier", which are used interchangeably, describe a carrier or a diluent that does not cause significant irritation to the subject and does not abrogate the biological activity and properties of the VB-201.

As used herein, the term "carrier" refers to a diluent, adjuvant, excipient, or vehicle with which the therapeutic is administered.

Herein the term "excipient" refers to an inert substance added to a pharmaceutical composition to further facilitate administration of an active ingredient.

A pharmaceutical composition unit dosage form may be formulated readily by combining VB-201 with pharmaceutically acceptable carriers well known in the art. Using such carriers the active ingredient (VB-201) is formulated, for example, as sachets, pills, caplets, capsules, tablets, dragee-cores or discrete (e.g., separately packaged) units of powder, granules, or suspensions or solutions in water or non-aqueous media. Thickeners, diluents, flavorings, dispersing aids, emulsifiers or binders may be desirable.

Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredient may be dissolved or suspended in suitable liquids, such as fatty oils, liquid paraffin, or liquid polyethylene glycols. In addition, stabilizers may be added. Formulations for oral administration may further include a protective coating, aimed at protecting or slowing enzymatic degradation of the preparation in the GI tract.

Composition unit dosage forms may, if desired, be presented in a pack or dispenser device, such as an FDA (the U.S. Food and Drug Administration) approved kit, which may contain one or more unit dosage forms containing VB-201. The pack or dispenser device may, for example, comprise metal or plastic foil, such as, but not limited to a blister pack. The pack or dispenser device may be accompanied by instructions for administration. The pack may also be accompanied by a notice associated with the container in a form prescribed by a governmental agency regulating the manufacture, use or sale of pharmaceuticals, which notice is reflective of approval by the agency of the form of the compositions for human administration. Such notice, for example, may be of labeling approved by the U.S. Food and Drug Administration for prescription drugs or of an approved product insert. Composition unit dosage forms comprising VB-201 formulated in a compatible pharmaceutical carrier may also be prepared, placed in an appropriate container, and labeled for treatment of an inflammatory disease or disorder, as defined herein. The inflammatory disease or disorder may be associated with an endogenous oxidized lipid.

As used herein, the phrase "an endogenous oxidized lipid" refers to one or more oxidized lipids that are present or formed *in vivo,* as a result of inflammatory and other cell- or humoral-mediated processes. Oxidized low-density lipoprotein (oxidized-LDL) is an example of an endogenous oxidized lipid associated with an inflammatory disease or disorder.

Representative inflammatory diseases and disorders treatable include, for example, idiopathic inflammatory diseases or disorders, chronic inflammatory diseases or disorders, acute inflammatory diseases or disorders, autoimmune diseases or disorders, infectious diseases or disorders, inflammatory malignant diseases or disorders, inflammatory transplantation-related diseases or disorders, inflammatory degenerative diseases or disorders, diseases or disorders associated with a hypersensitivity, inflammatory cardiovascular diseases or disorders (e.g., as described herein), inflammatory cerebrovascular diseases or disorders, peripheral vascular diseases or disorders, inflammatory glandular diseases or disorders, inflammatory gastrointestinal diseases or disorders, inflammatory cutaneous diseases or disorders, inflammatory hepatic diseases or disorders, inflammatory neurological diseases or disorders, inflammatory musculo-skeletal diseases or disorders, inflammatory renal diseases or disorders, inflammatory reproductive diseases or disorders, inflammatory systemic diseases or disorders, inflammatory connective tissue diseases or disorders, inflammatory tumors, necrosis, inflammatory implant-related diseases or disorders, inflammatory aging processes, immunodeficiency diseases or disorders, proliferative diseases and disorders and inflammatory pulmonary diseases or disorders, as is detailed hereinbelow.

Non-limiting examples of hypersensitivities include Type I hypersensitivity, Type II hypersensitivity, Type III hypersensitivity, Type IV hypersensitivity, immediate hypersensitivity, antibody mediated hypersensitivity, immune complex mediated hypersensitivity, T lymphocyte mediated hypersensitivity, delayed type hypersensitivity, helper T lymphocyte mediated hypersensitivity, cytotoxic T lymphocyte mediated hypersensitivity, TH1 lymphocyte mediated hypersensitivity, and TH2 lymphocyte mediated hypersensitivity.

Non-limiting examples of cerebrovascular diseases or disorders include stroke, cerebrovascular inflammation, cerebral hemorrhage and vertebral arterial insufficiency.

Non-limiting examples of peripheral vascular diseases or disorders include gangrene, diabetic vasculopathy, ischemic bowel disease, thrombosis, diabetic retinopathy and diabetic nephropathy.

Non-limiting examples of autoimmune diseases or disorders include all of the diseases caused by an immune response such as an autoantibody or cell-mediated immunity to an autoantigen and the like. Representative examples are chronic rheumatoid arthritis, juvenile rheumatoid arthritis, systemic lupus erythematosus, scleroderma, mixed connective tissue disease, polyarteritis nodosa, polymyositis/dermatomyositis, Sjogren's syndrome, Bechet's disease, multiple sclerosis, autoimmune diabetes, Hashimoto's disease, psoriasis, primary myxedema, pernicious anemia, myasthenia gravis, chronic active hepatitis , autoimmune hemolytic anemia, idiopathic thrombocytopenic purpura, uveitis, vasculitides and heparin induced thrombocytopenia.

Non-limiting examples of inflammatory glandular diseases or disorders include pancreatic diseases or disorders, Type I diabetes, thyroid diseases or disorders, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome.

Non-limiting examples of inflammatory gastrointestinal diseases or disorders include colitis, ileitis, Crohn's disease, chronic inflammatory intestinal disease, inflammatory bowel syndrome, inflammatory bowel disease, celiac disease, ulcerative colitis, an ulcer, a skin ulcer, a bed sore, a gastric ulcer, a peptic ulcer, a buccal ulcer, a nasopharyngeal ulcer, an esophageal ulcer, a duodenal ulcer and a gastrointestinal ulcer.

Non-limiting examples of inflammatory cutaneous diseases or disorders include acne, an autoimmune bullous skin disease, pemphigus vulgaris, bullous pemphigoid, pemphigus foliaceus, contact dermatitis and drug eruption.

Non-limiting examples of inflammatory hepatic diseases or disorders include autoimmune hepatitis, hepatic cirrhosis, and biliary cirrhosis.

Non-limiting examples of inflammatory neurological diseases or disorders include multiple sclerosis, Alzheimer's disease, Parkinson's disease, myasthenia gravis, motor neuropathy, Guillain-Barre syndrome, autoimmune neuropathy, Lambert-Eaton myasthenic syndrome, paraneoplastic neurological disease or disorder, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, Huntington's disease, AIDS associated dementia, amyotrophic lateral sclerosis (AML), multiple sclerosis, stroke, an inflammatory retinal disease or disorder, an inflammatory ocular disease or disorder, optic neuritis, spongiform encephalopathy, migraine, headache, cluster headache, and stiff-man syndrome.

Non-limiting examples of inflammatory connective tissue diseases or disorders include autoimmune myositis, primary Sjogren's syndrome, smooth muscle autoimmune disease or disorder, myositis, tendinitis, a ligament inflammation, chondritis, a joint inflammation, a synovial inflammation, carpal tunnel syndrome, arthritis, rheumatoid arthritis, osteoarthritis, ankylosing spondylitis, a skeletal inflammation, an autoimmune ear disease or disorder, and an autoimmune disease or disorder of the inner ear.

Non-limiting examples of inflammatory renal diseases or disorders include autoimmune interstitial nephritis and/or renal cancer.

Non-limiting examples of inflammatory reproductive diseases or disorders include repeated fetal loss, ovarian cyst, or a menstruation associated disease or disorder.

Non-limiting examples of inflammatory systemic diseases or disorders include systemic lupus erythematosus, systemic sclerosis, septic shock, toxic shock syndrome, and cachexia.

Non-limiting examples of infectious disease or disorder include chronic infectious diseases or disorders, a subacute infectious disease or disorder, an acute infectious disease or disorder, a viral disease or disorder, a bacterial disease or disorder, a protozoan disease or disorder, a parasitic disease or disorder, a fungal disease or disorder, a mycoplasma disease or disorder, gangrene, sepsis, a prion disease or disorder, influenza, tuberculosis, malaria, acquired immunodeficiency syndrome, and severe acute respiratory syndrome.

Non-limiting examples of inflammatory transplantation-related diseases or disorders include graft rejection, chronic graft rejection, subacute graft rejection, acute graft rejection hyperacute graft rejection, and graft versus host disease or disorder. Exemplary implants include a prosthetic implant, a breast implant, a silicone implant, a dental implant, a penile implant, a cardiac implant, an artificial joint, a bone fracture repair device, a bone replacement implant, a drug delivery implant, a catheter, a pacemaker, an artificial heart, an artificial heart valve, a drug release implant, an electrode, and a respirator tube.

Non-limiting examples of inflammatory tumors include a malignant tumor, a benign tumor, a solid tumor, a metastatic tumor and a non-solid tumor.

Non-limiting examples of inflammatory pulmonary diseases or disorders include asthma, allergic asthma, emphysema, chronic obstructive pulmonary disease or disorder, sarcoidosis and bronchitis.

An example of a proliferative disease or disorder is cancer.

Optionally, the inflammatory disease or disorder is a disease or disorder characterized in the medical art as being treatable by glatiramer acetate. Optionally, the inflammatory disease or disorder is selected from the group consisting of multiple sclerosis, colitis, inflammation associated with malaria (e.g., cerebral malaria) and dry age-related macular degeneration.

In all of the methods and treatment regimens described herein, VB-201 is optionally administered orally. The dosage of VB-201 is optionally in a range of from 1 μg/day to 1 gram/day. Optionally, the administered dosage of VB-201 is a therapeutic amount described in U.S. Provisional Patent Application Nos. 61/292,226 and 61/282,250, and in PCT International Patent Application entitled "TREATMENT WITH VB-201", having attorney's Docket No. 50377, which is co-filed with the instant application.

Thus, for example, the administered dosage of VB-201 may be, for example, 10 mg, 20 mg, 30 mg, 40 mg, 50 mg, 60 mg, 70 mg, 80 mg, 90 mg or 100 mg VB-201 per day.

There is described a pharmaceutical being packaged in a packaging material and identified, in or on the packaging material, for use according to a method described herein.

The pharmaceutical composition may be identified for use in combination with glatiramer acetate, for the treatment of an inflammatory disease or disorder (e.g., as described herein).

Suitable techniques for formulating a pharmaceutical composition comprising a therapeutically effective amount of VB-201 are described in International Patent Application No. PCT/IL2004/000453 (Publication No. WO 04/106486).

The pharmaceutical composition may be formulated for oral administration of VB-201.

There are described methods and pharmaceutical compositions as described herein, comprising an oxidized phospholipid other than VB-201. Suitable oxidized phospholipids, which are structurally related to VB-201 and thus exhibit similar activities, are described, for example, in International Patent Applications PCT/IL2004/000453 and PCT/IL2009/001049 (Publication Nos. WO 2004/106486 and WO 2010/052718, respectively).

Also, enantiomer, diastereomer, pharmaceutically acceptable salts, hydrates and solvates of the compounds (e.g., VB-201 and other oxidized phospholipids) described hereinabove may be used. VB-201 (1-hexadecyl-2-(4'-carboxybutyl)-glycerol-3-phosphocholine) may be a chiral enantiomer of 1-hexadecyl-2-(4'-carboxybutyl)-glycerol-3-phosphocholine, i.e., either the *(R)*- enantiomer (*(R)*-1-hexadecyl-2-(4'-carboxybutyl)-*sn*-glycerol-3-phosphocholine) or the *(S)*- enantiomer (*(S)*-1-hexadecyl-2-(4'-carboxybutyl)-glycerol-3-phosphocholine), or a mixture thereof (e.g., a racemate). VB-201 may be (R)-1-hexadecyl-2-(4'-carboxybutyl)-sn-glycerol-3-phosphocholine

According to the present disclosure, the term "prodrug" refers to an agent, which is converted into the active compound (the active parent drug) *in vivo.* Prodrugs are typically useful for facilitating the administration of the parent drug. They may, for instance, be bioavailable by oral administration whereas the parent drug is not. The prodrug may also have improved solubility as compared with the parent drug in pharmaceutical compositions. Prodrugs are also often used to achieve a sustained release of the active compound *in vivo.* An example, without limitation, of a prodrug would be a compound as described herein, having one or more carboxylic acid moieties, which is administered as an ester (the "prodrug"). Such a prodrug is hydrolysed *in vivo,* to thereby provide the free compound (the parent drug). The selected ester may affect both the solubility characteristics and the hydrolysis rate of the prodrug.

The phrase "pharmaceutically acceptable salt" refers to a charged species of the parent compound and its counter ion, which is typically used to modify the solubility characteristics of the parent compound and/or to reduce any significant irritation to an organism by the parent compound, while not abrogating the biological activity and properties of the administered compound. An example, without limitation, of a pharmaceutically acceptable salt would be a carboxylate anion and a cation such as, but not limited to, ammonium, sodium, potassium and the like.

The term "solvate" refers to a complex of variable stoichiometry (e.g., di-, tri-, tetra-, penta-, hexa-, and so on), which is formed by a solute (the compounds described herein) and a solvent, whereby the solvent does not interfere with the biological activity of the solute. Suitable solvents include, for example, ethanol, acetic acid and the like.

The term "hydrate" refers to a solvate, as defined hereinabove, where the solvent is water.

As used herein the term "about" refers to ± 10 %.

The terms "comprises", "comprising", "includes", "including", "having" and their conjugates mean "including but not limited to".

The term "consisting of means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

The description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

### MATERIALS AND METHODS

#### Materials:

Atorvastatin was obtained from Pfizer.
Ethanol was obtained from Bio Lab.
PBS (Dulbecco's phosphate buffered saline) was obtained from Biological Industries
VB-201 was dissolved at a concentration of 2.7 mg/ml in phosphate buffered saline (PBS) (Biological Industries, Israel) with 0.5 % ethanol (Bio-Lab, Israel).

#### Measurement of aortic lesion area:

Aortas were collected upon sacrifice. Lesion area was determined by scanning the intensity of Sudan IV stain adhering to the lesioned tissue, using Image-Pro Plus software (Media Cybernetics). Lesion areas were calculated in three different sections of the aorta (aortic arch, thoracic aorta and abdominal aorta). The total aortic lesion area (sum of lesion areas in all 3 sections) was calculated as a percentage of the sum of all 3 section areas.

#### Plasma cholesterol assay:

Plasma cholesterol levels were determined in the Bert W. Strassburger Lipid Center (Sheba Medical Center, Israel) using a Cobas Mira analyzer (Roche) and cholesterol reagent (Roche/Hitach).

#### Blood chemistry assay:

Blood chemistry was assayed by the Pathological Chemistry Institute Laboratory (Sheba Medical Center, Israel) using an AU2700® spectrophotometer (Olympus) and the appropriate Olympus reagents. The parameters evaluated were urea, creatinine, glucose, potassium, sodium, chloride, total calcium, phopshorus, uric acid, bilirubin, SGOT, SGPT, LDH, total CPK, CPK-MB, CK-MB %, total protein, albumin, globulin, alkaline phosphatase and osmolality levels.

#### Safety evaluation:

Safety and tolerability of VB-201 regimens are judged by evaluating the incidence of abnormal findings in the following measurements:

vital signs: systolic and diastolic blood pressure, pulse rate, respiratory rate, body temperature;
physical examination;
12-lead electrocardiogram;
laboratory tests of blood samples for: hemoglobin, hematocrit, mean corpuscular volume, mean corpuscular hemoglobin concentration, red blood cell count, white blood cell count, white blood cell differential count, platelet count, neutrophil count, LDL- and HDL-cholesterol, total cholesterol, triglycerides, glucose, urea, sodium, potassium, creatinine, calcium, chloride, inorganic phosphate, creatinine phosphokinase, aspartate transaminase, alanine transaminase, γ-glutamyl transferase, alkaline phosphatase, total protein, albumin, total and indirect bilirubin;
routine urinalysis; and
all adverse events, whether or not considered serious, and whether or not considered related to the investigational agent, according to the criteria for Good Clinical Practice.

Adverse events are defined as being related to the treatment whenever there is no unreasonable temporal relationship between administration and onset of the adverse event, no biologically plausibly causal relationship between the treatment and the adverse event, and/or there is a clearly more likely alternative explanation for the adverse event.

## EXAMPLE 1

### Combined atorvastatin and VB-201 treatment for atherosclerosis

Atherosclerosis was induced in male New Zealand White rabbits (hsdIF:NZ, Harlan) by supplying the rabbits with a high-cholesterol diet (0.5 % w/w cholesterol) for 14 weeks. The rabbits were assigned to four treatment groups (7 or 8 animals per group): a) atorvastatin treatment; b) VB-201 treatment; c) combined atorvastatin + VB-201 treatment; and d) PBS with 0.5 % ethanol (control group).

Atorvastatin was administered by supplementing the diet with 50 mg/kg atorvastatin, which corresponds to a daily dose of approximately 2.5 mg/kg, based on a daily food consumption of approximately 125 grams per rabbit and an average weight of 2.5 kg.

VB-201 was administered daily (5 days per week) by oral gavage of the stock solution (2.7 mg/ml) at volume of 1.5 ml per kg body weight, corresponding to a dose of approximately 4 mg/kg.

At the end of 14 weeks, the rabbits were sacrificed, and the effects of the treatments were evaluated by measurement of aortic lesion areas and plasma cholesterol levels, and routine blood chemistry assessment (as described hereinabove). Plasma cholesterol levels were also determined after 5 weeks of the high-cholesterol diet.

As shown in Figure 1, atorvastatin treatment reduced aortic lesion areas by 33 % and VB-201 treatment reduced aortic lesion areas by 39 % relative to the control group, whereas combined atorvastatin + VB-201 treatment reduced aortic lesion areas by 53 % relative to the control group.

As shown in Table 1 below, atorvastatin reduced cholesterol levels considerably (either alone or in combination with VB-201), whereas VB-201 per se did not reduce cholesterol levels.

### Table 1: Average cholesterol levels following a high-cholesterol diet for 5 or 14 weeks

| | Cholesterol (mg/dL) (% of control) | |
| --- | --- | --- |
| | 5 weeks | 14 weeks |
| **Treatment** | | |
| **Atorvastatin (2.5 mg/kg)** | 580 (56 %) * | 1082 (64 %) ** |
| **VB-201 (4 mg/kg)** | 1303 (126 %) * | 2195 (131 %) * |
| **Atorvastatin (2.5 mg/kg) + VB-201 (4 mg/kg)** | 609 (59 %) ** | 1163 (69 %) ** |
| **Control** | 1032 (100 %) | 1679 (100 %) |

** statistically significant (relative to control);
* statistically non-significant (relative to control)

Taking both groups receiving atorvastatin together showed a positive significant ($p < 0.05$) correlation ($R^2 = 0.3$) between total plasma cholesterol levels and lesion area upon sacrifice, indicating that reduction of lesion area by atorvastatin was via reduction of cholesterol levels.

As shown in Table 2 below, atorvastatin increased SGPT (serum glutamic pyruvic transaminase) levels and alkaline phosphatase levels considerably, whether administered alone ($p < 0.05$ relative to control, for both SGPT and alkaline phosphatase levels) or with VB-201 ($p < 0.05$ relative to control, for SGPT levels), whereas VB-201 had no apparent effect on SGPT or alkaline phosphatase levels, and even appeared to attenuate the atorvastatin-induced increase in alkaline phosphatase levels.

### Table 2: Average SGPT and alkaline phosphatase levels following a high-cholesterol for 14 weeks

| Treatment | SGPT (IU/L) (% of control) | Alkaline Phosphatase (IU/L) (% of control) |
| --- | --- | --- |
| **Atorvastatin (2.5 mg/kg)** | 110.63 (235 %) | 104.63 (174 %) |
| **VB-201 (4 mg/kg)** | 45.13 (96 %) | 68.00 (113 %) |
| **Atorvastatin (2.5 mg/kg) + VB-201 (4 mg/kg)** | 120.43 (255 %) | 84.57 (141 %) |
| **Control** | 47.14 (100 %) | 1679 (100 %) |

The above results indicate that VB-201 surprisingly and synergistically enhances the ability of statins to inhibit lesion growth, even though VB-201 does not contribute to the reduction of cholesterol levels which is the therapeutic mechanism of statins.

The above results further indicate that VB-201 is safer than atorvastatin, and that coadministration of VB-201 with atorvastatin does not worsen the adverse side effects of atorvastatin but rather minimizes them.

## EXAMPLE 2

### Combined glatiramer acetate and VB-201 treatment in dextran sulfate sodium (DSS)-induced colitis model

Colitis was induced in mice with dextran sulfate sodium (DSS), to serve as a model of inflammatory disorders. A solution of 2 % DSS was administered in the drinking water of the mice on days 0-4, 19-23 and 32-36 of the experiment.

VB-201 was administered by oral gavage at daily doses of 0.04, 0.4 or 4 mg/kg, beginning 5 days prior to disease induction (i.e., first day of DSS administration). In an alternative treatment, 2 mg per mouse of glatiramer acetate was administered subcutaneously, daily from day 0. Each treatment group included 20 mice. As a negative control, 15 mice were administered vehicle (PBS with 0.5 % ethanol) by gavage beginning 5 days prior to disease induction. As positive controls, 8 mice were administered vehicle (PBS with 0.5 % ethanol) by gavage beginning 5 days prior to disease induction, without treatment with DSS, and 5 mice received no treatment at all. Mice were sacrificed on day 39, and their colon length and weight was determined.

Mice were scored according to a disease activity index (DAI), based on the average score for weight loss, stool consistency and bleeding, as follows:

| Score | Weight loss (%) | Stool consistency | Occult/gross bleeding |
|---|---|---|---|
| 0 | None | Normal | Normal |
| 1 | 1-5 | | |
| 2 | 5-10 | Loose | |
| 3 | 10-15 | | |
| 4 | >15 | Diarrhea | Gross bleeding |

As shown in Figure 2, glatiramer acetate treatment resulted in 100 % survival until day 23, with survival decreasing to below that of control mice by day 39, whereas VB-201 treatment at all tested doses resulted in survival of at least 80 % at day 39.

Similarly, as shown in Figure 3, glatiramer acetate reduced the DAI of mice until about day 20 (the acute phase), and did not reduce the DAI thereafter, whereas VB-201 at all tested doses reduced the DAI of mice after about day 20 (the chronic phase), without reducing the DAI beforehand.

In addition, VB-201 reduced body weight loss over the period of days 17-24, whereas glatiramer acetate reduced body weight loss over the period of days 8-22.

As shown in Figure 4, VB-201 partially reversed the DSS-induced shortening of the colon length at all tested doses, whereas glatiramer acetate was not effective. Interestingly, treatment with 0.04 mg/kg VB-201 was more effective than the higher doses of VB-201.

As shown in Figure 5, VB-201 at all tested doses partially reversed the DSS-induced increase in the colon weight/length ratios. Glatiramer acetate had a similar effect.

The above results indicate that glatiramer acetate and VB-201 have different beneficial effects which may complement each other.

The effect of VB-201 in combination with glatiramer acetate was therefore tested using the above DSS-induced colitis model. VB-201 was administered to one group by oral gavage at a daily doses of 0.4 mg/kg, beginning 5 days prior to disease induction. Glatiramer acetate was administered to a second group subcutaneously at a daily dose of 2 mg per mouse, from day 0. Another group received both VB-201 and glatiramer acetate as described above. Each treatment group included 20 mice. As controls, mice were administered vehicle (PBS with 0.5 % ethanol) by gavage beginning 5 days prior to disease induction, with (20 mice; negative control) or without (7 mice; positive control) DSS treatment.

As shown in Figure 6, VB-201 in combination with glatiramer acetate reversed the DSS-induced shortening of the colon length considerably more than did VB-201 or glatiramer acetate alone. The increase in colon length obtained with VB-201 + glatiramer acetate (relative to control mice with DSS-induced colitis) was greater than the sum of the increases in colon length obtained with VB-201 and glatiramer acetate alone.

The above results indicate that VB-201 and glatiramer acetate in combination exhibit a synergistic anti-inflammatory effect.

*EXAMPLE 3*

***Efficacy of VB-201 with statins in patients with elevated high sensitivisty C-reactive protein (hs-CRP) levels***

As shown in Example 1, treatment with VB-201 in combination with statins is particularly effective against atherosclerosis. In addition, VB-201 was previously shown to be effective at reducing C-reactive protein (CRP) levels, a marker for inflammation, in healthy humans. The efficacy of VB-201 at reducing inflammation in patients treated with statins is therefore investigated.

A randomized, double blind, Phase II study is performed in subjects with elevated CRP levels receiving VB-201 with concomitant statins compared to subjects receiving statins alone.

Subjects who have a CRP level (as determined by a high sensitivity CRP assay) between 2-10 mg/l on 2 separate tests, and who have been on a stable high dose of statin for at least 3 months, are selected. High doses of statins include ≥20 mg/day atorvastatin or ≥10 mg/day rosuvastatin or ≥40mg/day simvastatin.

After screening and establishment of a baseline, eligible subjects are randomly assigned to receive 5, 20, 40, 80, 120 or 240 mg per day VB-201, or placebo, for a period of 4 weeks. Doses are administered orally at breakfast time. All subjects continue to receive statins at the same dose utilized prior to study entry.

Efficacy of each dosage level of VB-201 relative to placebo is determined by measuring absolute and percent change from baseline for high sensitivity CRP assay values at weeks 2, 4 and 8.

Additional criteria include the change from baseline at weeks 2, 4 and 8 of levels of additional inflammatory biomarkers (e.g., IL-1$\beta$, IL-6, IL-12, IL-17, IL-22, IL-23, IFN-$\alpha$, IFN-$\gamma$, TNF-$\alpha$, MCP-1, MIP-1$\alpha$, MIP-1$\beta$, IL-12 p40, IL-12 p70, MPO, SAA and/or IL-8).

Safety of VB-201 administration is evaluated as described hereinabove by physical examination, incidence of adverse effects, vital signs, clinical chemistry, hematology, urinalysis and electrocardiograms.

Statistical comparisons are performed using a two-sided comparison with a 5 % level of significance.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. In addition, citation or identification of any reference in this application shall not be construed as an admission that such reference is available as prior art to the present invention. To the extent that section headings are used, they should not be construed as necessarily limiting.

**Claims**

1. VB-201, 1-hexadecyl-2-(4'-carboxybutyl)-glycerol-3-phosphocholine, for use in treating or preventing an inflammatory disease or disorder, wherein a therapeutically effective amount of VB-201 is to be orally administered to a human subject in need thereof, wherein the therapeutically effective amount of VB-201 is a daily dosage of 160 mg, wherein the daily dosage is effected by administering a unit dosage form comprising 40 mg or 80 mg VB-201 multiple times per day at different times during the day.

2. VB-201 for use of claim 1, wherein the daily dosage is effected by administering two unit dosage forms, each comprising 80 mg VB-201, at different times during the day.

3. VB-201 for use of claim 1 or 2, wherein the inflammatory disease or disorder is

(i) an inflammatory gastrointestinal disease or disorder selected from the group consisting of colitis, ileitis, Crohn's disease, chronic inflammatory intestinal disease, inflammatory bowel syndrome, celiac disease, an ulcer, a skin ulcer, a bed sore, a gastric ulcer, a peptic ulcer, a buccal ulcer, a nasopharyngeal ulcer, an esophageal ulcer, a duodenal ulcer, and a gastrointestinal ulcer,
(ii) an autoimmune disease or disorder selected from the group consisting of chronic rheumatoid arthritis, juvenile rheumatoid arthritis, systemic lupus erythematosus, scleroderma, mixed connective tissue disease, polyarteritis nodosa, polymyositis/dermatomyositis, Sjogren's syndrome, Behçet's disease, multiple sclerosis, autoimmune diabetes, Hashimoto's disease, primary myxedema, pernicious anemia, myasthenia gravis, chronic active hepatitis, autoimmune hemolytic anemia, idiopathic thrombocytopenic purpura, uveitis, vasculitides, and heparin induced thrombocytopenia,
(iii) an inflammatory cardiovascular disease or disorder selected from the group consisting of occlusive diseases or disorders, atherosclerosis, a cardiac valvular disease, stenosis, restenosis, in-stent-stenosis, myocardial infarction, coronary arterial disease, acute coronary syndromes, congestive heart failure, angina pectoris, myocardial ischemia, thrombosis, Wegener's granulomatosis, Takayasu's arteritis, Kawasaki syndrome, anti-factor VIII autoimmune disease or disorder, necrotizing small vessel vasculitis, microscopic polyangiitis, Churg and Strauss syndrome, pauci-immune focal necrotizing glomerulonephritis, crescentic glomerulonephritis, antiphospholipid syndrome, antibody induced heart failure, thrombocytopenic purpura, autoimmune hemolytic anemia, cardiac autoimmunity, Chagas' disease or disorder, and anti-helper T lymphocyte autoimmunity,

(iv) an inflammatory cerebrovascular disease or disorder selected from the group consisting of stroke, cerebrovascular inflammation, cerebral hemorrhage, and vertebral arterial insufficiency, or

(v) a peripheral vascular disease or disorder selected from the group consisting of gangrene, diabetic vasculopathy, ischemic bowel disease, thrombosis, diabetic retinopathy, and diabetic nephropathy.

4. VB-201 for use of claim 1 or 2, wherein the inflammatory disease or disorder is colitis.

5. VB-201 for use of claim 1 or 2, wherein the inflammatory cardiovascular disease or disorder is atherosclerosis.

6. VB-201 for use of claim 1 or 2, wherein the inflammatory cardiovascular disease or disorder is acute coronary syndrome.

7. VB-201 for use of claim 1 or 2, wherein the inflammatory cardiovascular disease or disorder is angina pectoris.

8. VB-201 for use of claim 1 or 2, wherein the inflammatory cerebrovascular disease or disorder is stroke.

9. VB-201 for use of any one of claims 1 to 8, wherein a therapeutically effective amount of glatiramer acetate is to be co-administered with VB-201.

10. VB-201 for use of claim 9, wherein the therapeutically effective amount of glatiramer acetate is in a range of from 2-200 mg/day.

11. VB-201 for use of claim 1, wherein the inflammatory disease or disorder is a proliferative disease or disorder.

12. VB-201 for use of claim 11, wherein the proliferative disease or disorder is cancer.

**Patentansprüche**

1. VB-201, 1-Hexadecyl-2-(4'-carboxybutyl)-glycerol-3-phosphocholin, zur Verwendung bei der Behandlung oder Vorbeugung einer entzündlichen Erkrankung oder Störung, wobei eine therapeutisch wirksame Menge von VB-201 oral an ein menschliches Individuum zu verabreichen ist, das dieses benötigt, wobei die therapeutisch wirksame Menge von VB-201 eine tägliche Dosierung von 160 mg ist, wobei die tägliche Dosierung durch Verabreichung einer Einheit einer Darreichungsform, die 40 mg oder 80 mg VB-201 umfasst, mehrere Male pro Tag zu unterschiedlichen Zeiten während des Tages erfolgt.

2. VB-201 zur Verwendung nach Anspruch 1, wobei die tägliche Dosierung durch die Verabreichung von zwei Einheiten der Darreichungsform, wobei jede 80 mg VB-201 umfasst, zu verschiedenen Zeiten während des Tages erfolgt.

3. VB-201 zur Verwendung nach Anspruch 1 oder 2, wobei die entzündliche Erkrankung oder Störung

(i) eine entzündliche Magen-Darm-Erkrankung oder -Störung ist, die ausgewählt ist aus der Gruppe bestehend aus Colitis, Ileitis, Morbus Crohn, chronisch-entzündlicher Darmerkrankung, entzündlicher Darmerkrankung, Zöliakie, einem Geschwür, einem Hautgeschwür, einem Dekubitus, einem Magengeschwür, einem peptischen Geschwür, einem bukkalen Geschwür, einem Nasopharynxgeschwür, einem Ösophagusgeschwür, einem Zwölffingerdarmgeschwür und einem Magen-Darm-Geschwür,

(ii) eine Autoimmunerkrankung oder Autoimmunstörung ist, die ausgewählt ist aus der Gruppe bestehend aus chronisch rheumatoider Arthritis, juveniler rheumatoider Arthritis, systemischem Lupus erythematodes, Sklerodermie, Mischkollagenose, Polyarteritis nodosa, Polymoysitis/Dermatomyositis, Sjögren-Syndrom, Morbus Behçet, Multipler Sklerose, autoimmunem Diabetes, Hashimoto-Syndrom, primärem Myxödem, perniziöser Anämie, Myasthenia gravis, chronisch aktiver Hepatitis, autoimmunhämolytischer Anämie, idiopathischer thrombozytopenischer Purpura, Uveitis, Vasculitiden und Heparininduzierter Thrombozytopenie,

(iii) eine entzündliche kardiovaskuläre Erkrankung oder Störung ist, die ausgewählt ist aus der Gruppe bestehend aus Verschlusskrankheiten oder Verschlussstörungen, Atherosklerose, einem Herzklappenfehler, Stenose, Restenose, In-Stent-Stenose, Herzinfarkt, Koronararterienerkrankung, akuten Koronarsyndromen, Herzinsuffizienz, Angina pectoris, Myokardischämie, Thrombose, Wegener-Granulomatose, Takayasu-Arteriitis, Kawasaki-Syndrom, Anti-Faktor-VIII-Autoimmunerkrankung oder Autoimmunstörung, nekrotisierende Vaskulitis der kleinen Gefäße, mikroskopischer Polyangiitis, Churg-Strauss-Syndrom, Pauci-immuner fokaler nekrotisierender Glomerulonephritis, halbmondförmiger Glomerulonephritis, Antiphospholipid-Syndrom, Antikörper-induziertem Herzversagen, thrombozytopenischer Purpura, autoimmunhämolytischer Anämie, kardialer Autoimmunität, Chagas-Krankheit oder Chagas-Störung und Anti-T-Helfer-Lymphozyt-Autoimmunität,

(iv) eine entzündliche zerebrovaskuläre Erkrankung oder Störung ist, die ausgewählt ist aus der Gruppe bestehend aus Schlaganfall, zerebrovaskulärer Entzündung, Hirnblutung, und vertebro-basilärer Insuffizienz, oder

(v) eine periphere vaskuläre Erkrankung oder Störung, die ausgewählt ist aus der Gruppe bestehend aus Gangrän, diabetischer Vaskulopathie, ischämischer Darmerkrankung, Thrombose, diabetischer Retinopathie und diabetischer Nephropathie.

**4.** VB-201 zur Verwendung nach Anspruch 1 oder 2, wobei die entzündliche Erkrankung oder Störung Colitis ist.

**5.** VB-201 zur Verwendung nach Anspruch 1 oder 2, wobei die entzündliche kardiovaskuläre Erkrankung oder Störung Atherosklerose ist.

**6.** VB-201 zur Verwendung nach Anspruch 1 oder 2, wobei die entzündliche kardiovaskuläre Erkrankung oder Störung akutes Koronar-Syndrom ist.

**7.** VB-201 zur Verwendung nach Anspruch 1 oder 2, wobei die entzündliche kardiovaskuläre Erkrankung oder Störung Angina pectoris ist.

**8.** VB-201 zur Verwendung nach Anspruch 1 oder 2, wobei die entzündliche zerebrovaskuläre Erkrankung oder Störung ein Schlaganfall ist.

**9.** VB-201 zur Verwendung nach einem der Ansprüche 1 bis 8, wobei eine therapeutisch wirksame Menge von Glatirameracetat zusammen mit VB-201 zu verabreichen ist.

**10.** VB-201 zur Verwendung nach Anspruch 9, wobei die therapeutisch wirksame Menge an Glatirameracetat in einem Bereich von 2 bis 200mg/Tag liegt.

**11.** VB-201 zur Verwendung nach Anspruch 1, wobei die entzündliche Erkrankung oder Störung eine proliferative Erkrankung oder Störung ist.

**12.** VB-201 zur Verwendung nach Anspruch 11, wobei die proliferative Erkrankung oder Störung Krebs ist.

**Revendications**

**1.** VB-201, 1-hexadécyl-2-(4'-carboxybutyl)-glycérol-3-phosphocholine, pour une utilisation dans le traitement ou la prévention d'une maladie ou d'un trouble inflammatoire, dans lequel une quantité thérapeutiquement efficace de VB-201 est administrée par voie orale à un sujet humain en ayant besoin, dans lequel la quantité thérapeutiquement efficace de VB-201 est un dosage quotidien de 160 mg, dans lequel le dosage quotidien est effectué par l'administration d'une forme galénique unitaire comprenant 40 mg ou 80 mg de VB-201 plusieurs fois par jour à des moments différents durant la journée.

**2.** VB-201 pour une utilisation selon la revendication 1, dans lequel le dosage quotidien est effectué par l'administration de deux formes galéniques unitaires, chacune comprenant 80 mg de VB-201, à des moments différents durant la journée.

**3.** VB-201 pour une utilisation selon la revendication 1 ou 2, dans lequel la maladie ou le trouble inflammatoire est

(i) une maladie ou un trouble gastro-intestinal inflammatoire choisi dans le groupe constitué de la colite, l'iléite, la maladie de Crohn, une maladie intestinale inflammatoire chronique, le syndrome de l'intestin inflammatoire, la maladie coeliaque, un ulcère, un ulcère cutané, une escarre, un ulcère gastrique, un ulcère peptique, un ulcère buccal, un ulcère nasopharyngé, un ulcère oesophagien, un ulcère duodénal, et un ulcère gastro-intestinal,

(ii) une maladie ou un trouble auto-immun choisi dans le groupe constitué de la polyarthrite rhumatoïde chronique, la polyarthrite rhumatoïde juvénile, le lupus érythémateux systémique, la sclérodermie, la connectivite mixte, la polyartérite noueuse, la polymyosite/dermatomyosite, le syndrome de Sjögren, la maladie de Behçet, la sclérose en plaques, le diabète auto-immun, la maladie de Hashimoto, le myxoedème primaire, l'anémie pernicieuse, la myasthénie grave, l'hépatite active chronique, l'anémie hémolytique auto-immune, le purpura thrombocytopénique idiopathique, l'uvéite, les vascularites, et la thrombocytopénie induite par l'héparine,

(iii) une maladie ou un trouble cardiovasculaire inflammatoire choisi dans le groupe constitué de maladies ou de troubles occlusifs, l'athérosclérose, une maladie valvulaire cardiaque, la sténose, la resténose, la sténose au sein d'un stent, l'infarctus du myocarde, la coronaropathie, les syndromes coronariens aigus, l'insuffisance cardiaque congestive, l'angine de poitrine, l'ischémie myocardique, la thrombose, la granulomatose de Wegener, l'artérite de Takayasu, le syndrome de Kawasaki, la maladie ou le trouble auto-immun anti-facteur VIII, la vascularite nécrosante des petits vaisseaux, la polyangéite microscopique, le syndrome de Churg et Strauss, la glomérulonéphrite nécrosante focale pauci-immune, la glomérulonéphrite à croissants, le syndrome des antiphospholipides, l'insuffisance cardiaque induite par des anticorps, le purpura thrombocytopénique, l'anémie hémolytique auto-immune, l'auto-immunité cardiaque, la maladie ou le trouble de Chagas, et l'auto-immunité anti-lymphocytes T auxiliaires,

(iv) une maladie ou un trouble cérébrovasculaire inflammatoire choisi dans le groupe constitué de l'accident vasculaire cérébral, l'inflammation cérébrovasculaire, l'hémorragie cérébrale, et l'insuffisance artérielle vertébrale, ou

(v) une maladie ou un trouble vasculaire périphérique choisi dans le groupe constitué de la gangrène, la vasculopathie diabétique, la maladie de l'intestin ischémique, la thrombose, la rétinopathie diabétique, et la néphropathie diabétique.

4. VB-201 pour une utilisation selon la revendication 1 ou 2, dans lequel la maladie ou le trouble inflammatoire est la colite.

5. VB-201 pour une utilisation selon la revendication 1 ou 2, dans lequel la maladie ou le trouble cardiovasculaire inflammatoire est l'athérosclérose.

6. VB-201 pour une utilisation selon la revendication 1 ou 2, dans lequel la maladie ou le trouble cardiovasculaire inflammatoire est le syndrome coronarien aigu.

7. VB-201 pour une utilisation selon la revendication 1 ou 2, dans lequel la maladie ou le trouble cardiovasculaire inflammatoire est l'angine de poitrine.

8. VB-201 pour une utilisation selon la revendication 1 ou 2, dans lequel la maladie ou le trouble cérébrovasculaire inflammatoire est l'accident vasculaire cérébral.

9. VB-201 pour une utilisation selon l'une quelconque des revendications 1 à 8, dans lequel une quantité thérapeutiquement efficace d'acétate de glatiramère est coadministrée avec le VB-201.

10. VB-201 pour une utilisation selon la revendication 9, dans lequel la quantité thérapeutiquement efficace d'acétate de glatiramère se situe dans une plage de 2 à 200 mg/jour.

11. VB-201 pour une utilisation selon la revendication 1, dans lequel la maladie ou le trouble inflammatoire est une maladie ou un trouble prolifératif.

12. VB-201 pour une utilisation selon la revendication 11, dans lequel la maladie ou le trouble prolifératif est le cancer.

**FIG. 1**

FIG. 2

FIG. 3

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IL 2004000453 W
- WO 04106486 A
- IL 0101080 W
- WO 0241827 A
- IL 05000735 W
- WO 06006161 A
- IL 0200005 W
- WO 02053092 A
- IL 08000013 W
- WO 08084472 A
- US 20080261865 A
- US 61292226 A
- US 61282250 B
- IL 2009001049 W
- WO 2004106486 A
- WO 2010052718 A

### Non-patent literature cited in the description

- *Lancet,* 2002, vol. 350, 7-22
- *Lancet,* 1995, vol. 333, 1301-1307
- *Lancet,* 1994, vol. 344, 1383-1389
- *Lancet,* 2003, vol. 361, 1149-1157
- *Lancet,* 2004, vol. 364, 685-696